# EUROPÄISCHE PATENTSCHRIFT

(11) **EP 0 339 460 B1**
(45) Veröffentlichungstag und Bekanntmachung des Hinweises auf die Patenterteilung: **17.02.1993**
(21) Anmeldenummer: 89107033.6
(22) Anmeldetag: 19.04.1989
(51) Int. Cl.: A61M 25/00

(54) **Medizinisches Einführungsbesteck**
Set of medical introduction instruments
Jeu d'instruments médicaux d'introduction

(30) Priorität: 29.04.1988 DE 3814618
(43) Veröffentlichungstag der Anmeldung: 02.11.1989
(73) Patentinhaber: Baumgart, Rainer, Dipl.-Ing. Dr. med., D-81479 München (DE); Küffer, Georg, Dr. med., D-80335 München (DE)
(72) Erfinder: Baumgart, Rainer, Dr. med., 8000 München 71 (DE)
(74) Vertreter: Finck, Dieter, Dr.Ing.

(56) Entgegenhaltungen:
- EP-A- 0 045 849
- FR-A- 2 182 666
- US-A- 4 610 671

## Beschreibung

Die Erfindung betrifft ein medizinisches Einführungsbesteck mit einer Innenhülse aus flexiblem Material, deren distaler Endabschnitt sich bei gleichbleibendem Innendurchmesser nach distal leicht konisch verjüngt, und mit einer dazu koaxialen Außenhülse aus flexiblem Material, deren proximaler Endabschnitt eine den Durchgang wenigstens der Innenhülse zulassende und das Innenlumen der Außenhülse bei herausgezogener Innenhülse vollständig verschließende Dichtung aufweist, wobei die Innenhülse und die Außenhülse axial zueinander verschieblich sind. Ein solches Einführungsbesteck ist aus der US-PS 44 30 081 bekannt.

Die Plazierung des Einführungsbestecks erfolgt meist in der sogenannten Seldinger-Technik, bei welcher zunächst z. B. die Arterie mit einer Metallkanüle punktiert wird, durch welche ein flexibler Führungsdraht in das Gefäßlumen vorgeschoben wird. Danach wird die Metallkanüle zurückgezogen. Auf den Führungsdraht wird dann das Einführungsbesteck, bestehend aus einer Innenhülse sowie aus der diese umschließenden Außenhülse, aufgeschoben. Dabei wird zuerst der sich nach distal konisch verjüngende Endabschnitt der Innenhülse durch die von der Metallkanüle gebildete Öffnung in das Gefäßlumen eingeführt und unter Aufweisung dieser Öffnung so weit vorgeschoben, bis sich auch die Außenhülse ausreichend weit im Gefäß befindet.

Das Innenlumen der Außenhülse ist am proximalen Endabschnitt durch eine Dichtung abgedichtet, die z. B. von einer sternförmigen und von einer runden Gummilippe gebildet wird, die hintereinander angeordnet sind und die Innenhülse dicht umschließen, so daß auch dann, wenn die Innenhülse und der Führungsdraht aus der Außenhülse herausgezogen sind, kein Blut aus der Arterie austreten kann.

Die Außenhülse mit der proximalen Abdichtung wird auch als Kunststoffschleuse bezeichnet. Durch eine gut plazierte Schleuse lassen sich problemlos und schnell verschiedene Katheter, Endoskope oder andere Instrumentarien, wie zum Beispiel Biopsiezangen, durchführen, ohne daß bei jedem Wechsel die Punktionsumgebung geschädigt wird.

Die auf die Innenhülse aufgeschobene Außenhülse bildet, bezogen auf den Außendurchmesser der Innenhülse, eine ringförmige Außenschulter, was aufgrund der dünnen Wandstärke der Außenhülse beim Führen durch die Gefäßöffnung zu einem Aufstülpen und Einreißen des distalen Endabschnitts der Außenhülse und als Folge zu Gefäßwandläsionen bzw. zu umfangreichen Gewebeschäden längs des Stichkanals bei der Punktion von parenchymatösen Organen führen kann, wenn dieser Schaden nicht gleich erkannt wird. Nicht selten kommt es dadurch zu einem Blutaustritt bei liegender Schleuse oder zu Nachblutungen und in Einzelfällen bei Gefäßpunktionen auch zu narbigen Strikturen, wenn das Gefäßlumen einen Einriß erleidet.

Der Außendurchmesser der Außenhülse ist in seiner Größe durch die Maßgabe begrenzt, daß beim Entfernen der Schleuse ein Verschluß der perkutanen Punktionsöffnung ohne operative Maßnahmen erfolgen soll, bei spielsweise bei einer Arterie durch Kompression von ca. 30 bis 60 Minuten. Bei zunehmendem Außendurchmesser nimmt zudem die Wandstärke der Außenhülse, d.h. die Stufe in Einführungsrichtung und damit die oben beschriebene Gefährdung zu.

Die der Erfindung zugrunde liegende Aufgabe besteht nun darin, das Einführungsbesteck der gattungsgemäßen Art so zu verbessern, daß es bei Vorhandensein einer möglichst dünnwandigen Außenhülse mit großem Innenlumen in Einführungsrichtung stufenfrei ausgebildet ist, und daß sich die innerhalb der Außenhülse befindlichen Hülsen problemfrei entfernen lassen.

Diese Aufgabe wird ausgehend von dem Einführungsbesteck der eingangs genannten Art dadurch gelöst, daß der distale Endabschnitt der Innenhülse eine proximale Außenschulter aufweist. Zwischen der Außenhülse und der Innenhülse ist koaxial eine Zwischenhülse aus flexiblem Material angeordnet, die relativ zu der Innenhülse und zu der Außenhülse zwischen einer in die Außenhülse eingezogenen Stellung und einer ausgeschobenen Stellung verschiebbar ist. In der eingezogenen Stellung der Zwischenhülse liegt einerseits die Stirnseite des distalen Endabschnitts der Außenhülse an der proximalen Außenschulter der Innenhülse an. Andererseits ist der Außendurchmesser der Stirnseite des distalen Endabschnitts der Außenhülse genauso groß wie oder kleiner als der Außendurchmesser der proximalen Außenschulter der Innenhülse. In der ausgeschobenen Stellung der Zwischenhülse ist die Stirnseite ihres distalen Endabschnitts zur Stirnseite des durch die Zwischenhülse elastisch etwas aufgeweiteten distalen Endabschnitts der Außenhülse fluchtend ausgerichtet oder steht nach distal vor. Der Außendurchmesser der Außenschulter der Innenhülse ist genauso groß wie oder ist kleiner als der Außendurchmesser der Zwischenhülse.

Wenn das so aufgebaute Einführungsbesteck auf den z. B. in ein Gefäßlumen eingebrachten Führungsdraht aufgeschoben und durch die Öffnung in das Gefäßlumen vorgeschoben wird, hat das Einführungsbesteck außenseitig keine Abstufung in Einführungsrichtung, da die Stirnfläche der Außenhülse in Einschieberichtung durch die proximale Außenschulter des distalen Endabschnitts der Innenhülse abgedeckt ist. Die Öffnung in der Gefäßwand wird dadurch beim Einschieben des Bestecks stufenlos aufgeweitet, wodurch sowohl Schäden an dem distalen Endabschnitt der Außenhülse als auch an der Gefäßwand ausgeschlossen werden.

Wenn das Einführungsbesteck plaziert ist, müssen die Innenhülse und die Zwischenhülse gemeinsam mit dem Führungsdraht aus der von der Außenhülse mit der proximalen Abdichtung gebildeten Schleuse entfernt werden, um den Arbeitskanal für die Kathetermanipulationen freizugeben. Hierzu werden zunächst die Innenhülse und die Zwischenhülse um den gleichen Abstand weiter in das Gefäß vorgeschoben, wodurch der distale Endabschnitt der Zwischenhülse aus dem distalen Endabschnitt der Außenhülse herausgeschoben und dadurch die Außenhülse elastisch aufgeweitet wird, so daß ihr Innendurchmesser dem Außendurchmesser der Zwischenhülse entspricht. Der distale Endabschnitt der Innenhülse wird nun durch Zug am proximalen Ende von außerhalb bündig bis an die distale Stirnfläche der Zwischenhülse zurückgezogen und kann so das aufgeweitete distale Ende der Außenhülse bei weiterem Zug nach proximal passieren. Die Innenhülse kann nun gemeinsam mit der Zwischenhülse entfernt werden. Die Dichtung am proximalen Ende der Außenhülse verhindert Blut- bzw. Flüssigkeitsaustritt und ermöglicht den problemlosen Katheterwechsel. Nach Beendigung der Manipulationen kann die Schleuse einfach gezogen und die Punktionsöffnung durch entsprechende Kompression über eine vorgegebene Zeit geschlossen gehalten werden, bis sich die Öffnung ohne operative Maßnahmen von selbst geschlossen hat.

Bevorzugt weist die Zwischenhülse an ihrem distalen Endabschnitt innenseitig axial eine sich über den ganzen Innenumfang ersteckende, in einer Innenschulter endende Ausnehmung auf, während der Außendurchmesser der proximalen Außenschulter der Innenhülse genauso groß ist wie oder kleiner ist als der Innendurchmesser der Ausnehmung.

Bei dieser Ausgestaltung kann der distale Endabschnitt der Innenhülse in der distalen Ausnehmung der Zwischenhülse so weit aufgenommen werden, bis ihre Außenschulter an der Innenschulter der Zwischenhülse anliegt.

Wenn am proximalen Endabschnitt der Innenhülse und der Außenhülse jeweils ein Griff angebracht ist, können die erforderlichen Verschiebebewegungen einfach bewirkt werden.

Vorteilhafterweise ist eine einerseits am proximalen Endabschnitt der Innenhülse und am proximalen Endabschnitt der Zwischenhülse sowie andererseits am proximalen Endabschnitt der Außenhülse anliegende, entfernbare Distanzhülse vorgesehen. Dadurch ist beim Einführen des Einführungsbestecks gewährleistet, daß die distale Stirnseite der Außenhülse an der proximalen Außenschulter des distalen Endes der Innenhülse bündig anliegt und dadurch abgedeckt wird. Die Distanzhülse wird durch Aufklappen entfernt, wenn bei plaziertem Einführungsbesteck die Innenhülse und die Zwischenhülse aus der Außenhülse in der vorstehend beschriebenen Weise herausgezogen werden soll.

Zweckmäßigerweise werden eine Ringnut im proximalen Endabschnitt der Außenhülse und ein aus der Außenfläche der Zwischenhülse vorspringender, elastisch verformbarer Ringwulst vorgesehen, der in die Ringnut in der ausgeschobenen Stellung der Zwischenhülse eingreift. Dadurch wird sichergestellt, daß dann, wenn die Innenhülse und die Zwischenhülse aus der Außenhülse herausgezogen werden soll, die Innenhülse bündig an dem distalen Endabschnitt der Zwischenhülse anliegt. Dadurch wird beim Zurückziehen der Innenhülse die Zwischenhülse in der Außenhülse zunächst gehalten, bis die proximale Außenschulter des distalen Endabschnitts der Innenhülse außerhalb der Außenhülse fest an den Anschlag am distalen Ende der Zwischenhülse drückt und damit den Ringwulst der Zwischenhülse wieder aus der Ringnut der Außenhülse löst. Dadurch ist gewährleistet, daß die proximale Außenschulter der Innenhülse die distale Öffnung der Außenhülse sicher passieren kann, und daß sich die Innenhülse und die Zwischenhülse problemlos entfernen lassen. Bei dieser Anordnung folgt außerdem die Zwischenhülse nach Freigabe des Ringwulstes aus der Ringnut direkt der Bewegung der Innenhülse und bedarf keines zusätzlichen Griffs.

Das erfindungsgemäße medizinische Einführungsbesteck wird vorteilhaft bei Punktionen von Gefäßen, Körperhöhlen, Körperhohlorganen und parenchymatösen Organen, z. B. bei arteriellen, venösen, Blasen-, Pleura-, Gallengangs-, Uterus-, Peritoneal- oder Leberpunktionen verwendet.

Anhand von Zeichnungen wird ein Ausführungsbeispiel der Erfindung näher erläutet. Es zeigt:
Fig. 1 im Axialschnitt das Einführungsbesteck im Einführungszustand,
Fig. 2 in einer Ansicht wie Fig. 1 das Einführungsbesteck in einer Vorstufe für das Herausziehen der Innenhülse und der Zwischenhülse aus der Schleuse,
Fig. 3 in einer Ansicht wie Fig. 1 das Einführungsbesteck in dem Zustand, in welchem die Innenhülse und die Zwischenhülse aus der Schleuse herausgezogen werden können,
Fig. 4 im Axialschnitt eine abgeänderte Ausführung des distalen Endabschnitts der Zwischenhülse,
Fig. 5 perspektivisch die Handhabung des Einführungsbestecks beim Einführen über den liegenden Führungsdraht,
Fig. 6 perspektivisch die Handhabung für das Aufklappen der Distanzhülse,
Fig. 7 perspektivisch die Handhabung beim Verschieben der Innenhülse unter Mitnahme der Zwischenhülse und
Fig. 8 perspektivisch die Handhabung beim Herausziehen der Innenhülse unter Mitnahme der Zwischenhülse.

Das in der Zeichnung gezeigte Einführungsbesteck besteht aus drei schlauchförmigen Hülsen aus flexiblem Material, nämlich einer Außenhülse 30, einer Zwischenhülse 20 und einer Innenhülse 10, die koaxial aufeinandergeschoben und relativ zueinander in bestimmten axialen Abständen verschiebbar sind.

Die Innenhülse 10 hat an ihrem proximalen Endabschnitt 11 einen Griff 12. Der distale Endabschnitt 13 der Innenhülse 10 hat das gleiche Innenlumen 14 wie die übrige Innenhülse 10, jedoch eine gegenüber der übrigen Innenhülse 10 größere Wandstärke, wodurch proximal eine Außenschulter 15 gebildet wird, von der aus sich der distale Endabschnitt 13 zum Einführungsende hin verjüngt.

Die Zwischenhülse 20 hat einen proximalen zylinderförmigen Endabschnitt 21, dessen Außendurchmesser größer als der Außendurchmesser der Zwischenhülse 20 ist. Dieser Außendurchmesser der Zwischenhülse 20 ist mindestens so groß wie der Außendurchmesser der proximalen Außenschulter 15 des distalen Endabschnitts 13 der Innenhülse 10.

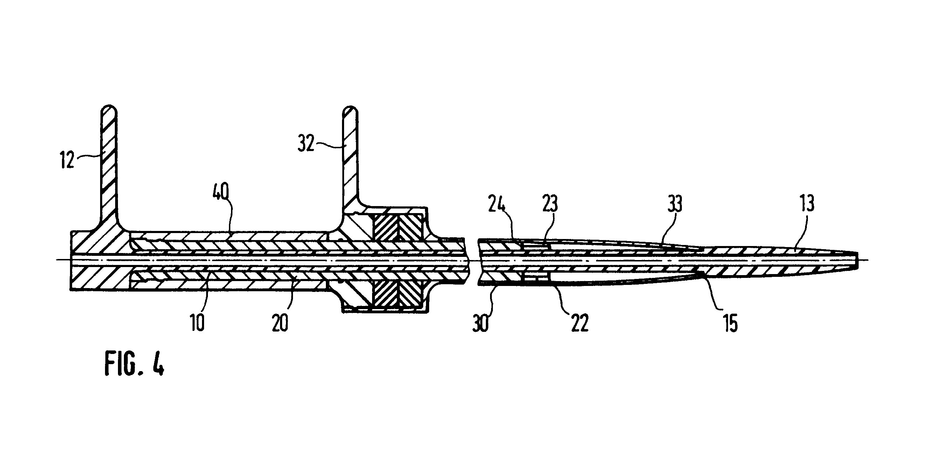

Bei der in Fig. 4 gezeigten Modifizierung ist in dem distalen Endabschnitt 22 der Zwischenhülse 20 von der Stirnseite her eine Ausnehmung 23 vorgesehen, die sich über die ganze Umfangsfläche erstreckt und in einer Innenschulter 24 endet. Der Innendurchmesser der Ausnehmung 23 ist etwas größer als der oder gleich dem Außendurchmesser des distalen Endabschnitts 13 der Innenhülse 10 im Bereich der Außenschulter 15 der Innenhülse 10, so daß der distale Endabschnitt 13 an der Seite der proximalen Außenschulter 15 in die Ausnehmung 23 eingeschoben werden kann.

Die Außenhülse 30 hat einen proximalen Abschnitt 31 mit einem Griff 32 und nimmt zwei Dichtungen 34 auf, von denen die eine sternförmig und die andere ringförmig ausgebildet ist und die meist aus Gummi hergestellt sind, der so elastisch verformbar ist, daß die Zwischenhülse 20 mit der Innenhülse 10 durch die Dichtungen 34 hindurchgeschoben werden kann, daß diese Dichtungen 34 jedoch das Innenlumen der Außenhülse 30 vollständig verschließen, wenn die Zwischenhülse 20 mit der Innenhülse 10 aus der Außenhülse 30 herausgezogen ist, um beispielsweise einen Katheter einführen zu können. Die Außenhülse 30 ist ausgehend von ihrem proximalen Abschnitt 31 dünnwandig ausgeführt und hat einen sich leicht konisch verjüngenden, jedoch radial elastisch aufweitbaren Endabschnitt 33, der an seiner distalen Stirnseite einen Außendurchmesser hat, der geringfügig kleiner ist oder genauso groß ist wie der Außendurchmesser des distalen Endabschnitts 13 der Innenhülse 10 im Bereich der proximalen Außenschulter 15.

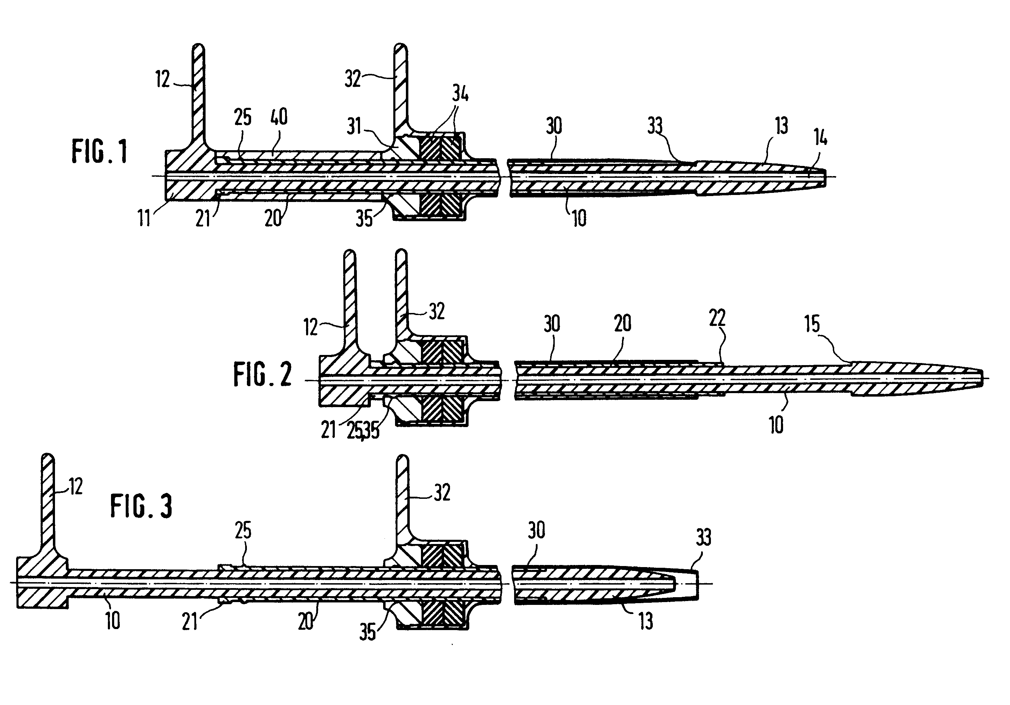

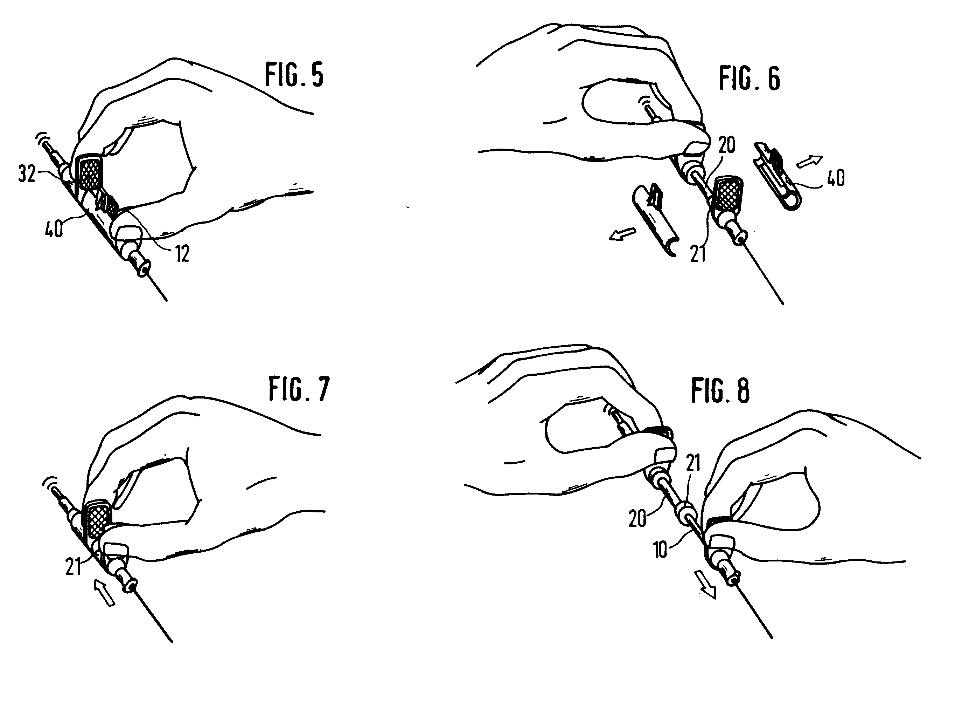

Wie aus Fig. 1 zu ersehen ist, liegt die Stirnseite des distalen Endabschnitts 33 der Außenhülse 30 an der proximalen Außenschulter 15 der verdickten distalen Endabschnitts 13 der Innenhülse 10 an, wobei der proximale Endabschnitt 11 der Innenhülse 10, der proximale Endabschnitt 21 der Zwischenhülse 20 und der proximale Endabschnitt 31 der Außenhülse 30 durch eine aufklappbare Distanzhülse 40 fixiert sind, wie dies in Fig. 5 gezeigt ist. In dieser Anordnung hat das Einführungsbesteck von seinem distalen Ende in Richtung seines proximalen Endes einen allmählich stufenlos zunehmenden Außendurchmesser, was das Einführen des Einführungsbestecks in eine Gefäßöffnung ermöglicht, ohne daß die dünnwandige Außenhülse 30 sich am distalen Ende aufstülpen oder einreißen kann.

In dem proximalen Endabschnitt 31 der Außenhülse 30 ist eine Ringnut 35 ausgespart. Von der Außenfläche der Zwischenhülse 20 steht ein Ringwulst 25 vor, der elastisch verformbar ist und in der ganz nach distal ausgeschobenen Stellung der Zwischenhülse 20 in die Ringnut 35 eingreift, was in Fig. 2 gezeigt ist.

Wenn nun die Innenhülse 10 und die Zwischenhülse 20 aus der Schleuse, also aus der Außenhülse 30 mit den Dichtungen 34, entfernt werden sollen, wird die Distanzhülse 40 durch Aufklappen entfernt, was in Fig. 6 gezeigt ist. Der proximale Endabschnitt 11 der Innenhülse 10 wird mit Hilfe des Griffs 12 zusammen mit dem anliegenden proximalen Endabschnitt 21 der Zwischenhülse 20 gegen den proximalen Endabschnitt 31 der Außenhülse 30 verschoben, bis die proximalen Endabschnitte 11 bzw. 21 der Innenhülse 10 bzw. der Zwischenhülse 20 am proximalen Endabschnitt 31 der Außenhülse 30 anliegen, was in Fig. 2 und Fig. 7 gezeigt ist. Am Ende dieser Verschiebung greift der Ringwulst 25 der Zwischenhülse 20 in die Ringnut 35 im proximalen Endabschnitt 31 der Außenhülse 30 ein. Ferner weitet bei dieser Verschiebung der distale Endabschnitt 22 der Zwischenhülse 20 den distalen Endabschnitt 33 der Außenhülse 30 auf, so daß der Außendurchmesser der Zwischenhülse 20 dem Innendurchmesser der Außenhülse 30 an deren distalen Endabschnitt 33 entspricht. Wie aus Fig. 2 zu ersehen ist, steht die Zwischenhülse 20 über die distale Stirnfläche der Außenhülse 30 vor.

Durch Wegziehen des Griffs 12 der Innenhülse 10 vom Griff 32 der Außenhülse 30 nach proximal wird zunächst der verdickte distale Endabschnitt 13 der Innenhülse 10 mit seiner proximalen Außenschulter 15 bündig an die distale Stirnseite der Zwischenhülse 20 gezogen. Bei der Modifizierung von Fig. 4 wird der distale Endabschnitt 13 der Innenhülse 10 in die Ausnehmung 23 der Zwischenhülse 20 gezogen, bis die Außenschulter 15 der Innenhülse 10 an der Innenschulter 24 der Zwischenhülse 20 anliegt.

Durch weiteren Zug am Griff 12 der Innenhülse 10 löst sich nach Überwindung eines geringen Widerstandes der Ringwulst 25 der Zwischenhülse 20 aus der Ringnut 35 des proximalen Endabschnitts 31 der Außenhülse 30. Die Zwischenhülse 20 folgt under Beibehaltung des Kontakts ihres distalen Endabschnitts 22 mit der proximalen Außenschulter 15 der Rückzugsbewegung der Innenhülse 10, was aus Fig. 8 zu ersehen ist, und gleitet in den distalen Endabschnitt 33 der Außenhülse 30, was in Fig. 3 gezeigt ist. Aus dieser Stellung können die Innenhülse 10 und die Zwischenhülse 20 ohne Schwierigkeiten aus der von der Außenhülse 30 und den Dichtungen 34 gebildeten Schleuse nach proximal herausgezogen werden.

## Patentansprüche

1. Medizinisches Einführungsbesteck mit einer Innenhülse (10) aus flexiblem Material, deren distaler Endabschnitt (13) sich bei gleichbleibendem Innendurchmesser nach distal leicht konisch verjüngt, und mit einer dazu koaxialen Außenhülse (30) aus flexiblem Material, deren proximaler Endabschnitt (31) eine den Durchgang wenigstens der Innenhülse (10) zulassende und das Innenlumen der Außenhülse (30) bei herausgezogener Innenhülse (10) vollständig verschließende Dichtung (34) aufweist, wobei die Innenhülse (10) und die Außenhülse (30) axial zueinander verschieblich sind, dadurch **gekennzeichnet**, daß der distale Endabschnitt (13) der Innenhülse (10) eine proximale Außenschulter (15) aufweist, daß zwischen der Außenhülse (30) und der Innenhülse (10) koaxial eine Zwischenhülse (20) aus flexiblem Material angeordnet ist, die relativ zu der Innenhülse (10) und zu der Außenhülse (30) zwischen einer in die Außenhülse (30) eingezogenen Stellung und einer ausgeschobenen Stellung verschiebbar ist,
daß in der eingezogenen Stellung der Zwischenhülse (20) einerseits die Stirnseite des distalen Endabschnitts (33) der Außenhülse (30) an der proximalen Außenschulter (15) der Innenhülse (10) anliegt und andererseits der Außendurchmesser der Stirnseite des distalen Endabschnitts (33) der Außenhülse (30) genauso groß ist wie oder kleiner ist als der Außendurchmesser der proximalen Außenschulter (15) der Innenhülse (10),
daß in der ausgeschobenen Stellung der Zwischenhülse (20) die Stirnseite ihres distalen Endabschnitts (22) zur Stirnseite des durch die Zwischenhülse (20) elastisch etwas aufgeweiteten distalen Endabschnitts (33) der Außenhülse (30) fluchtend ausgerichtet ist oder nach distal vorsteht, und
daß der Außendurchmesser der Außenschulter (15) der Innenhülse (10) genauso groß ist wie oder kleiner ist als der Außendurchmesser der Zwischenhülse (20).

2. Einführungsbesteck nach Anspruch 1, dadurch **gekennzeichnet**, daß die Zwischenhülse (20) an ihrem distalen Endabschnitt (22) innenseitig axial eine sich über den ganzen Innenumfang erstreckende, in einer Innenschulter (24) endende Ausnehmung (23) aufweist und daß der Außendurchmesser der proximalen Außenschulter (15) der Innenhülse (13) genauso groß ist wie oder kleiner ist als der Innendurchmesser der Ausnehmung (23).

3. Einführungsbesteck nach Anspruch 1 oder 2, dadurch **gekennzeichnet**, daß am proximalen Endabschnitt (11 bzw. 31) der Innenhülse (10) und der Außenhülse (30) jeweils ein Griff (12 bzw. 32) angebracht ist.

4. Einführungsbesteck nach einem der Ansprüche 1 bis 3, **gekennzeichnet** durch eine einerseits am proximalen Endabschnitt (11) der Innenhülse (10) und am proximalen Endabschnitt (21) der Zwischenhülse (20) sowie andererseits am proximalen Endabschnitt (31) der Außenhülse (30) anliegende, entfernbare Distanzhülse (40).

5. Einführungsbesteck nach einem der Ansprüch 1 bis 4, **gekennzeichnet** durch eine Ringnut (35) im proximalen Endabschnitt (31) der Außenhülse (30) und durch einen aus der Außenfläche der Zwischenhülse (20) vorspringenden, elastisch verformbaren Ringwulst (25), der in die Ringnut (35) in der ausgeschobenen Stellung der Zwischenhülse (20) eingreift.

## Claims

1. Set of medical insertion instruments with an inner sleeve (10) made of flexible material and of which the distal end section (13) tapers slightly conically towards the distal end whilst the inner diameter remains constant, and with an outer sleeve (30) of flexible material which is coaxial therewith and of which the proximal end section (31) comprises a seal (34) which leaves a passage for at least the inner sleeve (10) and completely seals the inner lumen of the outer sleeve (30) when the inner sleeve (10) is extracted, wherein the inner sleeve (10) and the outer sleeve (30) are axially displaceable relative to one another, characterised in that the distal end section (13) of the inner sleeve (10) comprises a proximal outer shoulder (15); in that an intermediate sleeve (20) of flexible material is disposed coaxially between the outer sleeve (30) and the inner sleeve (10) and is displaceable relative to the inner sleeve (10) and to the outer sleeve (30) between a position which is retracted into the outer sleeve (30) and an extended position; in that, in the retracted position of the intermediate sleeve (20) on the one hand, the end face of the distal end section (33) of the outer sleeve (30) abuts the proximal outer shoulder (15) of the inner sleeve (10) and, on the other hand, the outer diameter of the end face of the distal end section (33) of the outer sleeve (30) is as large as or smaller than the outer diameter of the proximal outer shoulder (15) of the inner sleeve (10); in that, in the extended position of the intermediate sleeve (20), the end face of the distal end section (22) thereof is aligned flush with the end face of the distal end section (33) of the outer sleeve (30) which is somewhat enlarged resiliently by means of the intermediate sleeve (20) or projects towards the distal end; and in that the outer diameter of the outer shoulder (15) of the inner sleeve (20) is as large as or smaller than the outer diameter of the intermediate sleeve (20).

2. Set of insertion instruments according to Claim 1, characterised in that, on its distal end section (22) the intermediate sleeve (20) comprises axially on the interior a recess (23) which extends over the entire inner periphery and terminates in an inner shoulder (24); and in that the outer diameter of the proximal outer shoulder (15) of the inner sleeve (13) is as large as or smaller than the inner diameter of the recess (23).

3. Set of insertion instruments according to Claim 1 or 2, characterised in that a gripping device (12, 32 respectively) is provided on the proximal end section (11, 31 respectively) of the inner sleeve (10) and the outer sleeve (30) in each case.

4. Set of insertion instruments according to any one of Claims 1 to 3, characterised by a removable spacer sleeve (40) which on the one hand abuts the proximal end section (11) of the inner sleeve (10) and the proximal end section (21) of the intermediate sleeve (20) and, on the other hand, the proximal end section (31) of the outer sleeve (30).

5. Set of insertion instruments according to any one of Claims 1 to 4, characterised by an annular groove (35) in the proximal end section (31) of the outer sleeve (30) and by a resiliently deformable annular bead (25) which projects out of the outer surface of the intermediate sleeve (20) and engages the annular groove (35) in the extended position of the intermediate sleeve (20).

## Revendications

1. Dispositif d'injection médical comportant une gaine intérieure (10) en matériau souple, dont l'extrémité distale (13) s'effile, tout en conservant un diamètre intérieur constant, ayant une forme légèrement conique vers l'extrémité distale, et comportant une gaine extérieure (30) en matériau souple, coaxiale à ladite gaine (10), dont l'extrémité proximale (31) est munie d'un joint (34) permettant le passage d'au moins la gaine intérieure (10) et obturant totalement le volume intérieur de la gaine extérieure (30) lorsque la gaine intérieure (10) est sortie, la gaine intérieure (10) et la gaine extérieure (30) pouvant se déplacer axialement l'une par rapport à l'autre, caractérisé en ce
que l'extrémité distale (13) de la gaine intérieure (10) présente un épaulement extérieur proximal (15),
qu'entre la gaine extérieure (30) et la gaine intérieure (10) est disposée, coaxialement, une gaine intermédiaire (20) en matériau souple pouvant se déplacer, par rapport à la gaine intérieure (10) et la gaine extérieure (30), entre une position avancée et une position retirée dans la gaine extérieure (30),
que, en position avancée de la gaine intermédiaire (20), d'une part, le côté frontal de l'extrémité distale (33) est voisine de l'épaulement extérieur proximal (15) de la gaine intérieure (10) et, d'autre part, le diamètre extérieur du bord frontal (33) de l'extrémité distale de la gaine extérieure (30) est exactement égal ou inférieur au diamètre extérieur de l'épaulement extérieur proximal (15) de la gaine intérieure (10),
que, en position retirée de la gaine intermédiaire (20), le côté frontal (22) de l'extrémité distale est alignée avec le bord frontal du tronçon de l'extrémité distale, quelque peu élargi élastiquement par la gaine intermédiaire (20), de la gaine extérieure (30) ou est en saillie vers l'extrémité distale par rapport audit bord, et
que le diamètre extérieur de l'épaulement extérieur (15) de la gaine intérieure (10) est exactement égal ou inférieur au diamètre extérieur de la gaine intermédiaire (20).

2. Dispositif d'injection suivant la revendication 1, caractérisé en ce que, dans son tronçon d'extrémité distale (22), la gaine intermédiaire (20) présente axialement, du côte intérieur, un évidement (23) s'étendant sur toute la périphérie intérieure et aboutissant dans un épaulement intérieur (24) et que le diamètre extérieur de l'épaulement extérieur proximal (15) de la gaine intérieure (13) est exactement égal ou inférieur au diamètre intérieur de l'évidement (23).

3. Dispositif d'injection suivant la revendication 1 ou 2, caractérisé en ce que sur l'extrémité proximale (11, 31 respectivement) de la gaine intérieure (10) et de la gaine extérieure (30) est placée une poignée (12, 32 respectivement).

4. Dispositif d'injection suivant l'une des revendications 1 à 3, caractérisé par une gaine d'écartement (40) amovible, d'une part, contigüe à l'extrémité proximale (11) de la gaine intérieure (10) et à l'extrémité proximale (21) de la gaine intermédiaire (20) et, également, contigüe à l'extrémité proximale (31) de la gaine extérieure (30).

5. Dispositif d'injection suivant l'une des revendications 1 à 4, caractérisé par une rainure annulaire (35) dans le tronçon d'extrémité proximale (31 ) de la gaine extérieure (30) et par un bourrelet annulaire (25) déformable élastiquement, en saillie par rapport à la face extérieure de la gaine intermédiaire (20), qui, en position retirée de la gaine intermédiaire (20), pénètre dans la rainure annulaire (35).
